# EUROPÄISCHE PATENTSCHRIFT

(11) **EP 3 432 951 B1**
(45) Veröffentlichungstag und Bekanntmachung des Hinweises auf die Patenterteilung: **30.09.2020**
(21) Anmeldenummer: 17729017.8
(22) Anmeldetag: 23.03.2017
(51) Int. Cl.: A61M 11/00, A61F 9/00, A61M 5/24, A61M 5/28, B05B 11/02

(54) **KARTUSCHENTROPFENDOSIERER**
DROP DOSING CARTRIDGE
CARTOUCHE DE DOSAGE DE GOUTTES

(30) Priorität: 23.03.2016 DE 102016105508; 04.08.2016 DE 102016114405
(43) Veröffentlichungstag der Anmeldung: 30.01.2019
(73) Patentinhaber: F+K Innovationen GmbH & Co.KG, 76534 Baden-Baden (DE)
(72) Erfinder: FUCHS, Karl-Heinz, 78315 Radolfzell (DE)
(74) Vertreter: Patentanwälte und Rechtsanwalt Weiß, Arat & Partner mbB
(86) Internationale Anmeldenummer: PCT/EP2017/056954
(87) Internationale Veröffentlichungsnummer: WO 2017/162805

(56) Entgegenhaltungen:
- US-A- 3 166 070
- US-A- 4 344 573
- US-A- 5 944 702
- US-A1- 2007 257 063
- US-A1- 2010 065 049

## Beschreibung

### Technisches Gebiet

Die Erfindung betrifft ein Kartuschentropfendosierer nach dem Oberbegriff des Anspruchs 1.

### Stand der Technik

Aus dem Stand der Technik sind Multitropfendosierer als Systeme bekannt, welche auf Behältnisse wie Flaschen aufgesetzt werden.
In diesem Zusammenhang wird auf die US 5 944 702 A verwiesen, welche eine Vorrichtung zum Verabreichen eines Fluids durch eine Düse, wie etwa eine Augenbehandlungsvorrichtung zum Verabreichen eines Medikaments oder anderer Substanzen auf ein Auge offenbart, wobei die Düse einen äußeren Düsenabschnitt eine Dicke aufweist, die von einer ersten Stelle in Richtung zur Spitze der Düse kontinuierlich abnimmt.

Die US 2007/257063 A1 beschreibt eine Vorrichtung zum dosierten Ausbringen eines Mediums aus einem Behälter durch eine Öffnung in einem Gehäuseoberteil, wobei der Öffnung zum Verschließen eine Ventilkolbenspitze zugeordnet ist, soll an die Ventilkolbenspitze als Teil eines Ventilkolbens eine sich gegen eine Feder abstützende Kolbenfläche anschließen, die in einem Kolbenraum in dem Gehäuseoberteil abdichtend geführt ist und zwischen sich und der Öffnung einen Füllraum ausbildet, der mit dem Behälter in Verbindung steht.

Ausserdem zeigt die US 4 344 573 A einen Sprühapplikator für flüssige oder pulverförmige Arzneimittel zur einmaligen Anwendung, der eine genaue Dosierung des Arzneimittels in den Nasalbereich oder an anderen Körperstellen erlaubt.

Daneben wird auf die US 3 166 070 A und die US 2010/065049 A1 hingeweisen, welche verschiedene Spritzenkonstruktion offenbaren.

### Aufgabe der Erfindung

Die Aufgabe der Erfindung ist es ein Kartuschentropfendosierer als austauschbare Kartusche bereitzustellen, welche auf ein Betätigungselement vom Benutzer aufsetzbar ist. Dabei soll der Kartuschentropfendosierer einen Auslass aufweisen, welcher ein einfaches Ausbringen ermöglicht, wobei der Wiederverschluss des Auslasses automatisch erfolgt und den Innenraum der Kartusche wieder vollständig verschliesst.

### Lösung der Aufgabe

Zur Lösung der Aufgabe führen die Merkmale nach dem Anspruch 1. Vorteilhafte Ausgestaltungen sind in den Unteransprüchen beschrieben.

Die Idee besteht darin, dass das Lagern des flüssigen Medikamentes in einer austauschbaren Kartusche mit allen nötigen Funktionen wie:
Konservierungsstofffreie Lagerung, exakte Tropfengenerierung, kein verkeimbares, verschmutzbares Totvolumen, mechanisches druckgesteuertes Auslassventil in Verbindung mit einer Ausbringvolumen gesteuerten Betätigung und mit einem wieder verwendbaren Betätigungselement sowie über Wochen nach dem Verpackungsanbruch verwendbares Medikament, so dass das Augentropfenprodukt gefahrlos und wirkungsvoll appliziert werden kann.

Der von dem System erzeugte Tropfen soll sich von der Kartuschen-Abtropffläche in Birnenform lösen und keine verkeimbaren Flüssigkeitsreste auf der Abtropffläche hinterlassen.

Um den vom Auslassventil generierte Tropfen von der Abtropfgeometrie leicht abzulösen gibt das Ventil dem sich lösenden Tropfenvolumen eine leichte Beschleunigung aufgrund der doppelwandigen Ventilgeometrie.

Die auswechselbare doppelwandige Kartusche ist als längliches, zylindrisches Element aufgebaut, wobei in der zentralen Achse das Flüssigkeitsreservoir angeordnet ist, welches im offenen Abfüllbereich der Kartusche durch einen verschiebbaren Stopfen nach dem Abfüllen der Flüssigkeit verschlossen wird.

Im Device gegenüber des Stopfens ist die Auslassventilgeometrie als Begrenzung der Kartusche integriert.

Das Tropfenauslass- und Tropfenbildungsventil ist als Schlauchventil konzipiert, wobei der zentrische Mittelpin als Verlängerung der Wandung des Flüssigkeitsreservoirs als Ventilträger fungiert.

Bei dem Schlauchventil handelt es sich um eine Ventilbauart, wie sie auch bei den bekannten Dosierpumpen für den Pharma- und Kosmetikbereich als totvolumenfreies und verkeimungsfreies Auslassventil zum Einsatz kommen kann und die auf dem Markt befindlichen Kugelauslassventile, Druckdifferenzauslassventile und ferdergesteuerte Auslassventile ersetzen kann.

Über den Mittelpin ist eine zweite flexible Wandung in Form einer zylindrischen Verkleidung komplett über die gesamte Länge der Kartusche montiert, wobei am unteren Ende der Verkleidung der Zapfen für die Fixierung der Kartusche im Betätigungselement angeordnet ist.

Durch die flexible und formgebundene Wandung der Verkleidung im oberen Ventilbereich wird die Öffnung- und Schließkraft des Ventils gesteuert und eingestellt.

Die Ventilfunktion wird erreicht, indem im Mittelpin eine zentrale Längsbohrung verbunden mit einer Querbohrung zum Außendurchmesser des Pins integriert ist. Oder an dem Mitelpin Außendurchmesser eine oder mehrere Längsnuten die Flüssigkeit vom zentralen Flüssigkeitsreservoir zwischen die beiden Wandungen des Pins und der Verkleidung führt.

Durch das Verschieben des Stopfens über das Betätigungselement wird die Flüssigkeit im Reservoir über die Zuführungen der Bohrungen oder Nuten gegen die flexible Wandung der Verkleidung gedrückt, so dass die flexible Wandung sich aufdehnt.

Aufgrund unterschiedlicher Wandstärken und Geometrien und dadurch resultierenden erzeugten Anlagekräften der umlaufenden Wandung drängt sich das sich aufbauende Tropfenvolumen über den sich bildenden Ringspalt zwischen den beiden Wandungen zum Systemauslass.

Als zusätzliche Abdichtung können umlaufende Abdichtrillen am unteren Ende des Pins oder der Verkleidung vorgesehen werden.

Der Ventilauslass und die Abtropffläche ist eine Planfläche, welche durch die Planfläche des Pins sowie der Ringfläche der Verkleidung gestaltet wird.

Das Medium tritt in der geometrischen Trennung zwischen der Ringfläche und der Pinplanfläche in die Atmosphäre aus.

Durch die Eigenspannung der flexiblen Kunststoff-Verkleidungswandung bzw. Ventilwandung wird eine schnelle Funktionsreaktion des Ventils beim Schließen und Öffnen erreicht.

Ebenfalls wird durch die permanente spielfreie umlaufende unter Vorspannung stehende Verkleidungswandung zum Pindurchmesser eine garantierte Anlage der Verkleidungswandung garantiert, so dass keine Restflüssigkeit oder Luft in der Trennung der beiden Wandungen sich festsetzen und verkeimen kann, da auch keine Luft aus der Atmosphäre in das System eindringen kann.

Ebenfalls können keine Schmutzkeime von außen in das Ventil bzw. System hineinwachsen und dadurch gibt es dem Anwender die Sicherheit, immer ein sauberes Medikament zu applizieren.

Aufgrund der Geometrie der Abtropfplanfläche gibt es auch keine geometrischen Vertiefungen, Kanten, wo sich Restflüssigkeiten einnisten und verkeimen können.

Zum zusätzlichen Schutz während der Lagerung des Produktes kann die Kartusche mit einer Schutzkappe versehen werden.

Durch die Doppelwandung auch im Reservoirbereich wird die Diffusionsdichte sowie der Lichtschutzfaktor erhöht, somit können auch empfindlichere Flüssigkeiten ohne Konservierungsstoffe in das System abgefüllt, gelagert und zur Patientenanwendung kommen.

Die auswechselbare Kartusche wir über einen Bajonettverschluss mit dem wieder verwendbaren Betätigungselement zur Applikation lösbar verankert.

Das Betätigungselement mit einer Dreipunkt-Fingerauflage kann als Plattengeometrie oder auch als eine Komplettummantelung des als Perforation angespritzten Verzahnungskolbens ausgestaltet sein.

Ein Teilbereich des Betätigungselementes ist der verzahnte Kolben mit der Fingerauflage. Der Kolben ist gegenüber der Fingerauflage über eine Perforation mit dem restlichen Betätigungselementenkörper verbunden, wobei die Perforierung beim ersten Betätigen bricht und der Kolben über die Gegenverzahnung am Betätigungskörper zur unteren Planfläche des Systems der eingesetzten Kartusche geführt wird.

Die Verzahnung des Kolbens ist so aufgebaut, dass pro Zahnhöhe und Hub das Volumen eines Tropfens z.B. 30 µl im Reservoir zum Auslass der Kartusche verdrängt werden.

Der Anwender hört und spürt beim Betätigen des Kolbens über den Zahnratscheffekt, wenn er einen Tropfen zum Systemauslass gedrückt hat.

Durch die Anwendung am Auge kann der Patient nicht sehen, wenn ein Tropfen zur Verfügung steht, deshalb wird über den hörbaren und spürbaren Betätigungsablauf dem Anwender ebenfalls eine Handlingssicherheit geboten.

Um das Betätigungselement mit einer neuen Kartusche bestücken zu können, wird der in Endposition gedrückte Kolben nach der Entnahme der gebrauchten Kartusche in die Rückholstellung gedreht und in die Ausgangsstellung zurückgezogen.

Als Alternative zum ventilgeschlossenen System kann auch ein offenes System auf die gleiche Bauweise hergestellt werden, wobei das Auslassventil durch einen direkten Tropfenauslass ersetzt wird.

Um beim Alternativsystem die offene Kartusche bei der Lagerung vor der Anwendung gegen Verkeimung zu schützen, wird die im Gebrauch benötigte Schutzkappe als Produktschutz mit der geschlossenen Seite an der Kartuschenauslassseite über eine definierte Abreißkante mit angespritzt, so dass der Tropfenaustritt bis zur Applikation verschlossen ist.

Das heißt, vor dem ersten Gebrauch des Systems wird der Produktschutz manuell vom Anwender abgedreht und nach der Applikation als Schutzkappe aufgesetzt.

Durch das Abdrehen des Produktschutzes an der Auslassöffnung der Kartusche wird die Tropferöffnung für die Anwendung geöffnet.

Die Geometrie im Bereich der Auslassöffnung der Kartusche in Verbindung mit dem angespritzten Produktschutz ist ein zylindrischer, konischer Durchbruch mit einer dünnen, konisch verlaufenden umlaufenden Wandstärke für die gezielte manuelle Trennung des Produktschutzes.

Die vorliegende Erfindung mit dem Preservative -free Ventil wird dem Patient ein System ohne Konservierungsstoffe und ohne zusätzliches Silbernitrat zur Vermeidung der Systemverkeimung zur Verfügung stellen. Der Patient erhält zur Applikation das reine Medikament bei hoher Handlingssicherheit.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- **Fig. 1**: einen Querschnitt durch den erfindungsgemäßen Kartuschen-Tropfendosierer;
- **Fig. 2**: einen Querschnitt durch die erfindungsgemäße Kartusche;
- **Fig. 3**: einen Querschnitt durch den Ventilbereich der erfindungsgemäßen Kartusche;
- **Fig. 4**: einen Querschnitt durch ein Ausführungsbeispiel des Ventilbereiches der erfindungsgemäßen Kartusche;
- **Fig. 5**: eine Ansicht des offenen Systems mit aufgesetzter Schutzkappe;
- **Fig. 6**: einen Querschnitt durch den Anbindungsbereich des verzahnten Betätigungskolbens;
- **Fig. 7**: eine Ansicht der Kartusche mit angebundener Schutzkappe;
- **Fig. 8**: eine Ansicht des offenen Systems mit aufgesetzter Kartusche;
- **Fig. 9**: einen Querschnitt durch das offene System mit aufgesetzter Kartusche und aufgesteckter Schutzkappe;
- **Fig. 10**: einen Querschnitt durch den Tropfenauslass des offenen Systems;
- **Fig. 11**: einen Querschnitt durch ein weiters Ausführungsbeispiel des Schlauchventilbereiches auf einem handelsüblichen Pumpsystem;
- **Fig. 12**: einen Querschnitt durch ein weiters Ausführungsbeispiel des Schlauchventilbereichs auf einem handelsüblichen Pumpsystem;
- **Fig. 13**: einen Querschnitt durch ein weiters Ausführungsbeispiel des Schlauchventilbereiches auf einer Quetschflasche; und
- **Fig. 14**: einen Querschnitt durch ein weiters Ausführungsbeispiel des Schlauchventilbereiches auf einer Quetschflasche mit einem Innenbeutel.

### Ausführungsbeispiel

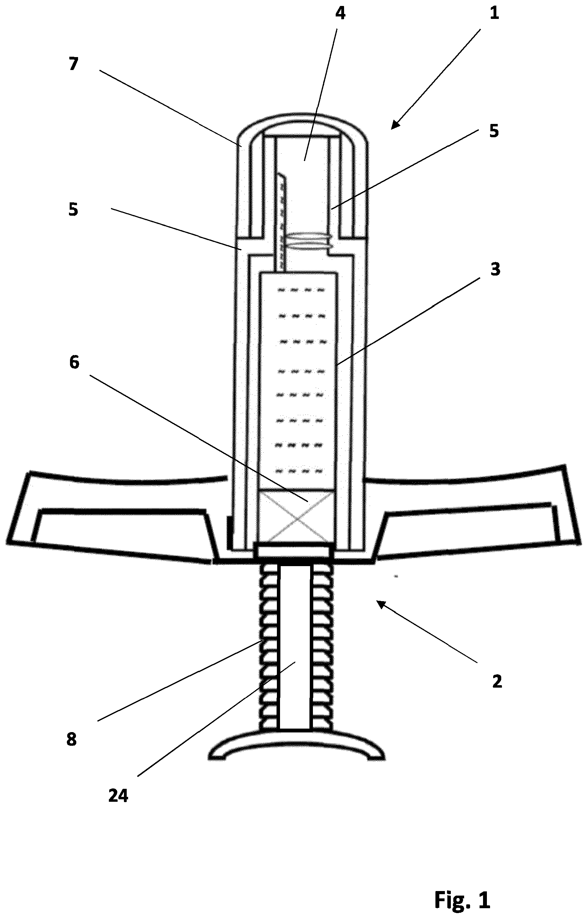

Figur 1 zeigt einen Querschnitt durch die Kartusche 1 und das Betätigungselement 2.

Die Kartusche 1 besteht aus dem Reservoirkörper 3 mit dem Ventilpin 4 sowie der Verkleidung 5, dem Stopfen 6 und der Schutzkappe 7.

Das Betätigungselement 2 ist einteilig mit dem perforiert angebundenen verzahnten Kolben 8 mit der Rückholnut 24.

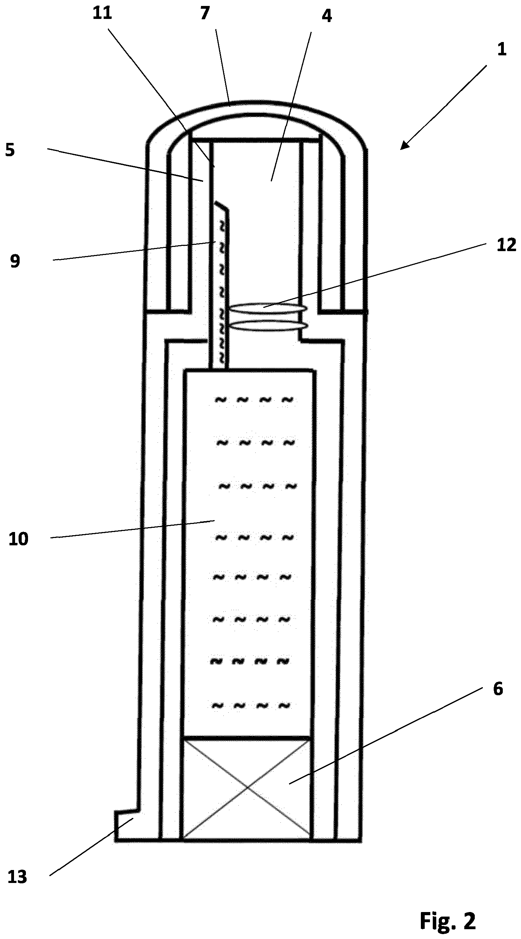

Figur 2 zeigt einen Querschnitt durch die Kartusche 1 mit Schutzkappe 7, zwischen der Verkleidungswandung 5 und dem Ventilpin 4 läuft die Flüssigkeitsnut 9 vom Reservoir 10 in den Schlauchventilbereich 11.

Als zusätzliche Flüssigkeitsdichtung sind umlaufenden Dichtrippen 12 um den Pindurchmesser vorgesehen.

Im Bereich des Stopfens 6 ist an der Verkleidungswand 5 der Verriegelungszapfen 13 zwischen der Kartusche 1 und Betätigungselement 2 angeordnet.

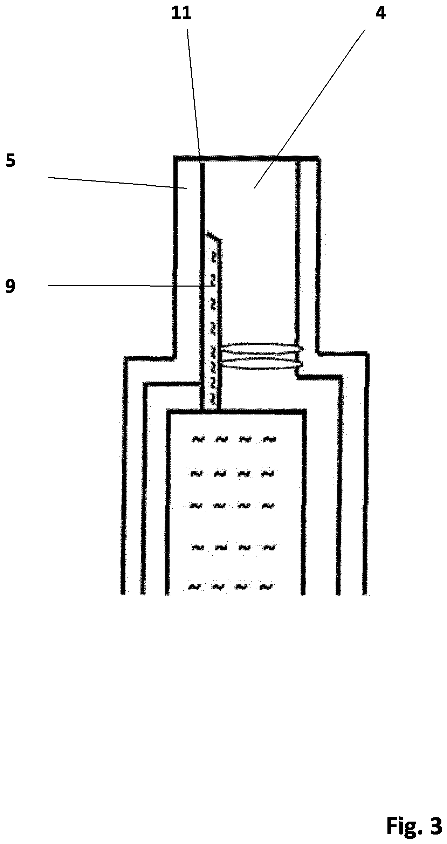

Figur 3 zeigt einen Querschnitt durch den Ventilbereich 11 der Kartusche 1.

Im Schlauchventil-Abdichtbereich 11 liegt die Verkleidungswandung 5 mit leichter Vorspannung auf den Pindurchmesser 4 satt an.

Die Flüssigkeitsnut 9 bringt das zu applizierende Medikament in den Abdichtbereich 11, so dass sich ein Ringspalt bildet und die Flüssigkeit austreten lässt.

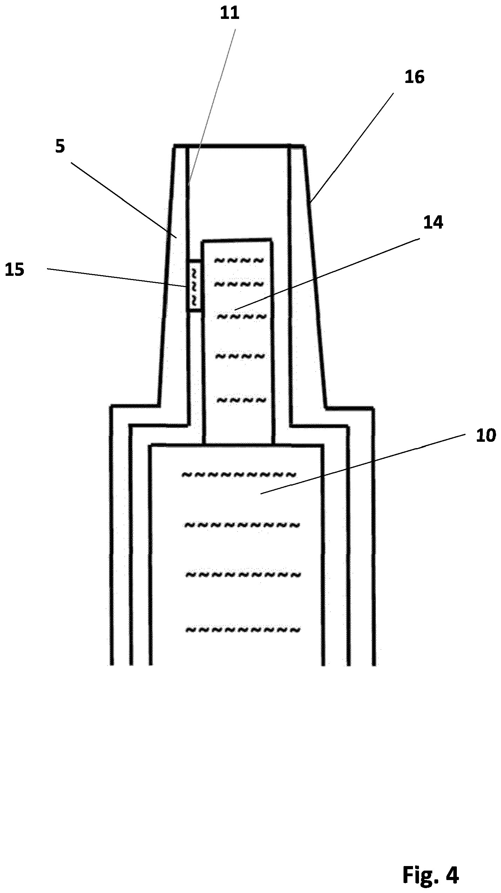

Figur 4 zeigt einen Querschnitt durch eine Variante des Ventilbereichs 11 der Kartusche 1.

Die zentrale Bohrung 14 versorgt die Querbohrung 15 aus dem Flüssigkeitsreservoir 10 mit dem auszubringenden Medikament, um es über den Abdichtbereich 11 als Tropfen aus der Kartusche 1 austreten zu lassen.

Mit der Verkleidungsgeometrie 16 der Verkleidung 5 werden die benötigten Dicht- und Öffnungskräfte des Schlauchventils erzeugt.

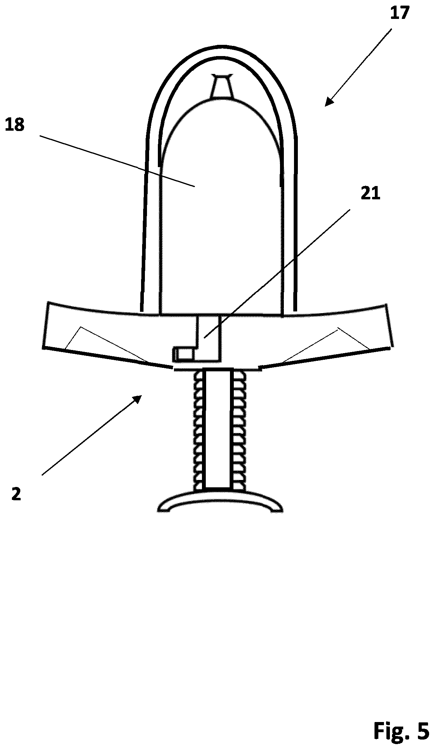

Figur 5 zeigt eine Ansicht des offenen Systems 17 mit eingesetzter offener Kartusche 18 und dem Betätigungselement 2 mit dem verzahnten Kolben 8.

Ebenfalls ist die Bajonettverriegelung 21 dargestellt.

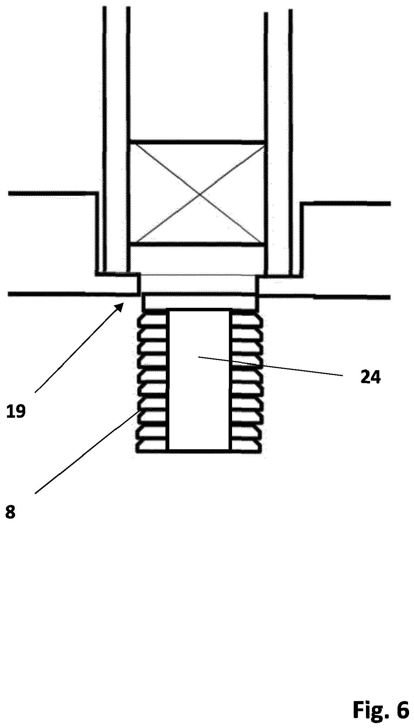

Figur 6 zeigt einen Querschnitt durch den Perforationsbereich 19 mit dem verzahnten Kolben 8 und der Rückholnut 24.

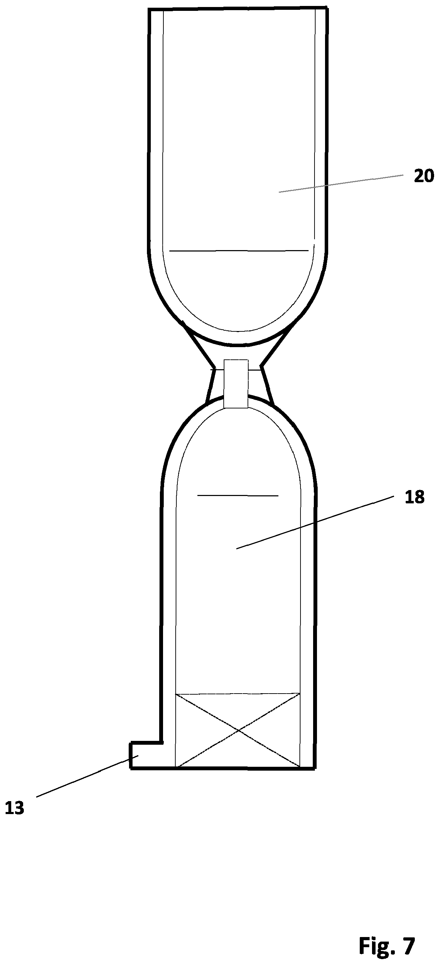

Figur 7 zeigt eine Ansicht der offenen Kartusche 18 mit angebundenem Produktschutz / Schutzkappe 20 und dem Verriegelungszapfen 13.

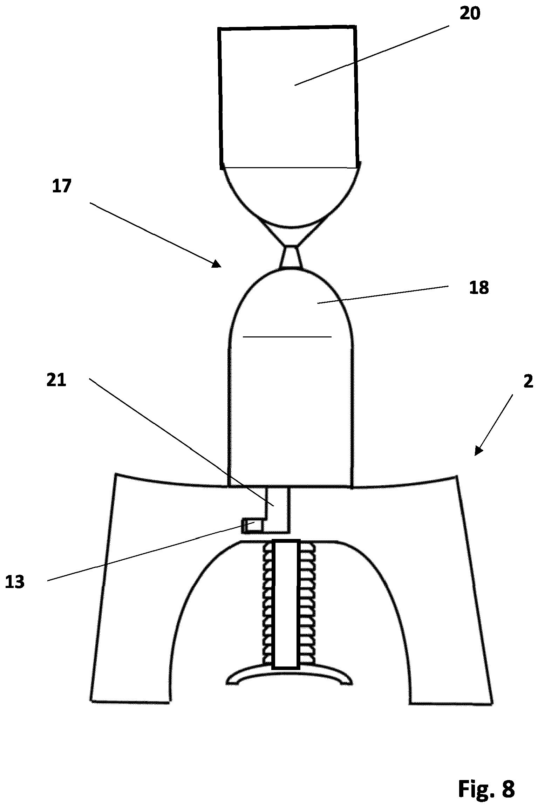

Figur 8 zeigt eine Ansicht und Variante des offenen Systems mit eingesetzter Kartusche 18 und noch nicht abgetrenntem Produktschutz / Schutzkappe 20 sowie eine geometrische Variante des Betätigungsschutzes 2 mit der Bajonettverriegelung 21 und dem Verriegelungszapfen 13.

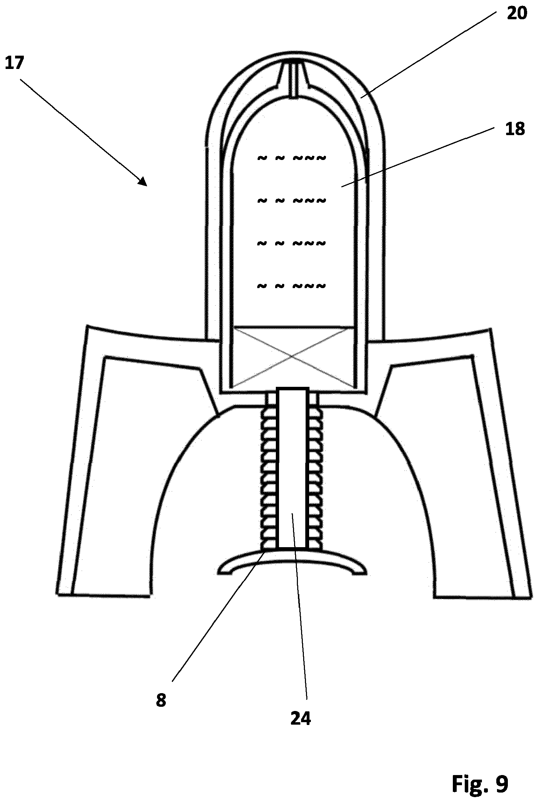

Figur 9 zeigt einen Querschnitt durch das offene System mit der geometrischen Ummantelungsvariante des Kolbens 8 im Betätigungselement 2 sowie der aufgesteckten Schutzkappe 20 auf der Kartusche 18.

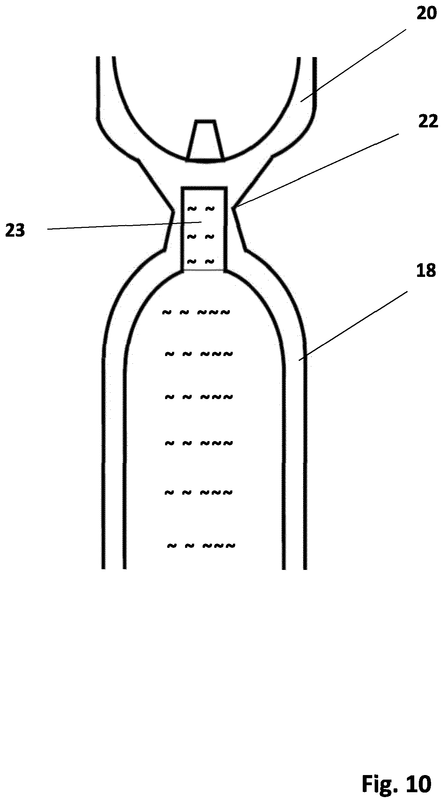

Figur 10 zeigt einen Querschnitt durch den Teilbereich der offenen Variante im Auslass der Kartusche 18 mit dem noch nicht abgetrennten Produktschutz / Schutzkappe 20. Der Abtrennbereich 22 mit der Trenngeometrie öffnet die Auslassbohrung 23 beim Entfernen des Produktschutzes, welcher dann als Schutzkappe 20 verwendet wird.

Weitere Ausführungsbeispiele sind derart gestaltet, dass der Schlauchventilbereich auf ein handelsübliches Pumpsystem oder direkt auf eine handelsübliche Quetschflasche gesetzt werden kann.

Der erfindungsgemäße Schlauchventilbereich weist weniger Teile auf als die aus dem Stand der Technik bekannten Pump- oder Quetschsystemauslassventile.

Das zweiteilige Ventilsystem mit dem adaptierten Pump- oder Quetschflaschensystem wirkt als dichtes nicht rücksaugendes Tropfendosierventil ohne verkeimbaren Totraum.

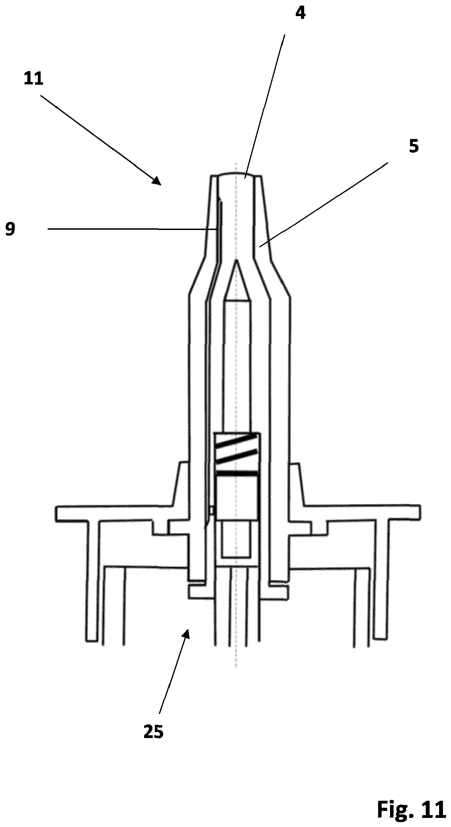

Fig.11 zeigt einen Querschnitt des Schlauchventilbereiches 11, den Ventilpin 4 mit der Flüssigkeitsnut 9 sowie der Verkleidung 5 auf einem handelsüblichen Pumpsystem.

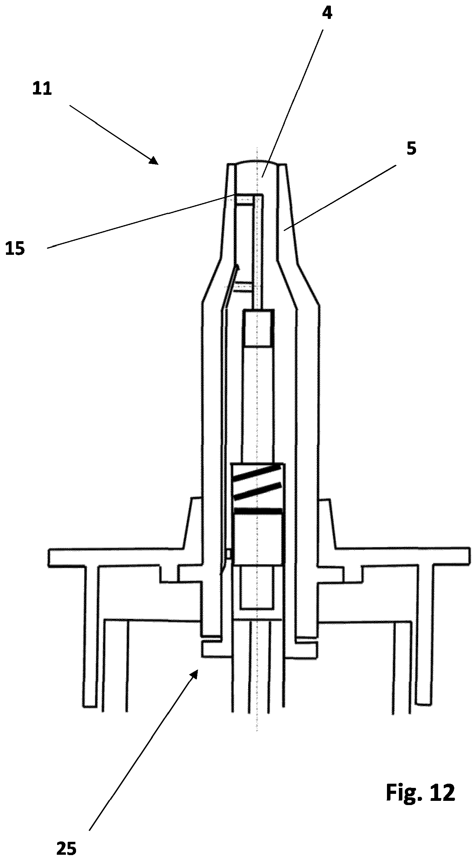

Fig.12 zeigt einen Querschnitt des Schlauchventilbereichs 11, den Ventilpin 4 mit der Querbohrung 15 sowie die Verkleidung 5 auf einem handelsüblichen Pumpsystem.

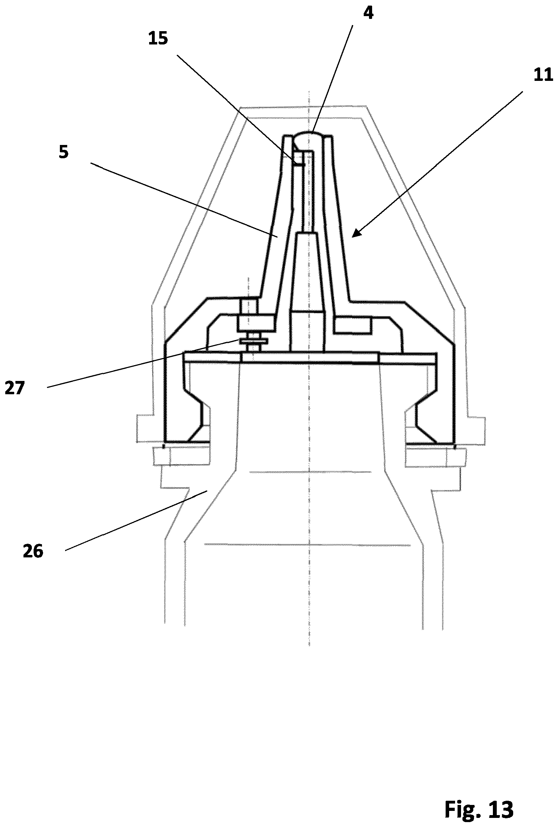

Fig.13 zeigt einen Querschnitt des Schlauchventilbereiches 11 auf einer Quetschflasche 26. Der Luftausgleich der Flasche wird über einen Filter 27 gesteuert. Die Verkleidung 5 hat an der Flaschenhalsseite einen Schnappverschluss und der Ventilpin 4 steht mit einem integrierten Flansch auf dem Flaschenhals.

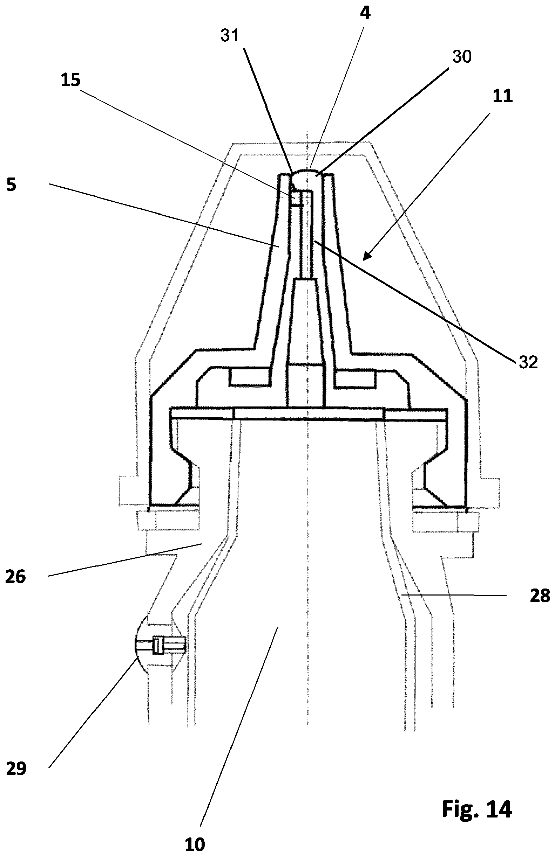

Fig.14 zeigt einen Querschnitt des Schlauchventilbereiches 11 auf einer Quetschflasche 26 mit einem Innenbeutel 28, die Verkleidung 5 hat an der Flaschenhalsseite einen Schnappverschluss zur Verankerung mit der Flasche und der Ventilpin 4 steht mit einem integrierten Flansch auf dem Flaschenhals in Wirkverbindung. Der Ventilpin 4 ist im Schlauchventilbereich 11 angeordnet. Dazu besteht er aus einem den Auslassbereich abdeckenden Teller 30, wobei der Teller 30 auf der der Querbohrung 15 gegenüberliegenden Seite eine Wölbung aufweist und diese Wölbung im Bereich der Querbohrung 15 eine Fase 31 aufweist.

Ausserdem umfasst der Teller 30 ein Verbindungsstück 32, welches sich vom Teller 30 hin zu der Quetschflasche 26 erstreckt, aber Teil des Schlauchventilbereichs 11 bleibt. Bei einem Ausbringen von Flüssigkeit aus der Querbohrung 15 wird die Fase 31 von der Flüssigkeit verdrängt und sobald der Druck der Flüssigkeit nachlässt, schliesst die Fase 31 augenblicklich den Auslassbereich.

Der Innenbeutel 28 hat bevorzugt eine geringere Versteifung oder Härte als die Quetschflasche 26, so dass durch das Plattenventil 29 einströmende Luft sich um den Innenbeutel 28 verteilen kann und dieser sich entsprechend der abnehmenden Menge an Flüssigkeit im Flüssigkeitsreservoir 10 zusammenzieht.

Das System arbeitet ohne Luftausgleich im Flüssigkeitsreservoir 10. Durch das entstehende Vakuum beim Quetschen der Flasche zieht sich der Innenbeutel 28 zusammen. Um über die Quetschflasche 26 den Innenbeutel 28 mit Druck zu beaufschlagen, strömt über ein in der Flaschenwandung integriertes Plattenventil 29 Luft oder Flüssigkeit zwischen Innenbeutel und Flaschenwandung. Das Plattenventil 29 funktioniert und schließt zur Atmosphäre, so dass nur Medium zwischen den Innenbeutel und Flaschenwandung einströmen und nicht ausströmen kann.

Die Dosiertropfengenerierung aus einem zur Umwelt dichten Flüssigkeitsreservoir 10 über den Schlauchventilbereich 11 in Verbindung mit einer Quetschflasche 26 sowie in der Flasche mit einem unter Vakuum zusammenziehbaren Innenbeutel 28 und in der Flaschenwandung integriertem Plattenventil 29 funktioniert über den Quetschdruck, ausgelöst über das Zusammendrücken der Flaschenwandung und dadurch entstehendem Druck auf den Innenbeutel 28.

Der sich durch die Tropfenvolumenentnahme bzw. Flüssigkeitsentnahme aus dem Innenbeutel 28 bildende Hohlraum zwischen Quetschflasche 26 und Innenbeutel 28 wird als Druckspeicher gegen den Innenbeutel 28 benutzt, wobei mit komprimierter Luft durch das automatische Verschließen des Plattenventils 29 oder mit Flüssigkeit gegen die Beutelwandung über das Quetschen der Flasche gedrückt wird.

Die Tropfengenerierung funktioniert auch mit Flüssigkeit im Hohlraum zwischen der Quetschflasche 26 und Innenbeutel 28 sowie unter Wasser bzw. in flüssigen Medien.

Das System mit dem Schlauchauslassventilbereich, der Verankerung zur Flasche und den Innenbeutel als Flüssigkeitsreservoir bleibt auch während der Applikation und Lagerung hundertprozentig dicht, so dass auch bei mehrwöchiger Anwendung kein Bakterienwachstum im System aufkommen kann.

### Bezugszeichenliste

| | |
|---|---|
| 1 | Kartusche |
| 2 | Betätigungselement |
| 3 | Reservoirkörper |
| 4 | Ventilpin |
| 5 | Verkleidung |
| 6 | Stopfen |
| 7 | Schutzkappe |
| 8 | Kolben |
| 9 | Flüssigkeitsnut |
| 10 | Reservoir |
| 11 | Schlauchventilbereich |
| 12 | Dichtrippen |
| 13 | Verriegelungszapfen |
| 14 | zentrale Bohrung |
| 15 | Querbohrung |
| 16 | Verkleidungsgeometrie |
| 17 | offenes System |
| 18 | offene Kartusche |
| 19 | Perforationsbereich |
| 20 | Produktschutz/Schutzkappe |
| 21 | Bajonettverriegelung |
| 22 | Abtrennbereich |
| 23 | Auslassbohrung |
| 24 | Rückholnut |
| 25 | Pumpsystem |
| 26 | Quetschflasche |
| 27 | Filter |
| 28 | Innenbeutel |
| 29 | Plattenventil |
| 30 | |
| 31 | |
| 32 | |
| 33 | |

## Patentansprüche

1. Kartuschentropfendosierer mit einer Kartusche (1) und einem Betätigungselement (2), wobei die Kartusche (1) einen Reservoirkörper (3) mit einem Ventilpin (4), eine Verkleidung (5) und einen Stopfen (6) umfasst, **gekennzeichnet durch**
umlaufende Dichtrippen (12) um einen Pindurchmessen sowie durch
eine Flüssigkeitsnut (9) zwischen einer Wandung der Verkleidung (5) und dem Ventilpin (4) von dem Reservoirkörper (3) in einen Schlauchventilbereich (11) hineinragt, wobei die Flüssigkeitsnut (9) entweder durch das Nachgeben der Dichtrippen (12) und/oder durch das Nachgeben eines Teils der Verkleidung (5) freigebbar ist.

2. Kartuschentropfendosierer nach Anspruch 1, **dadurch gekennzeichnet, dass** das Betätigungselement (2) einen Kolben (8) umfasst, wobei der Kolben (8) mit dem Stopfen (6) wirkverbindbar ist.

3. Kartuschentropfendosierer nach einem der Ansprüche 1 bis 2, **dadurch gekennzeichnet, dass** die umlaufenden Dichtrippen (12) und/oder die Verkleidung (5) druckflexibel und rückstellend ist.

4. Kartuschentropfendosierer nach einem der vorigen Ansprüche, **dadurch gekennzeichnet dass** die Kartusche einen Bajonettverschluss (13) umfasst, wobei der Bajonettverschluss (13) mit einer Bajonettverriegelung (21) des Betätigungselements (2) verbindbar ist.

5. Kartuschentropfendosierer nach einem der vorigen Ansprüche, **dadurch gekennzeichnet, dass** das Betätigungselement (2) einen Perforationsbereich (19) umfasst, wobei der Perforationsbereich (19) zwischen dem Kolben (8) und einem Gegenstück des Betätigungselements (2) angeordnet ist.

6. Kartuschentropfendosierer nach einem der vorigen Ansprüche, **dadurch gekennzeichnet, dass** der Kolben (8) eine Verzahnung aufweist.

7. Kartuschentropfendosierer nach einem der vorigen Ansprüchen, **dadurch gekennzeichnet, dass** die Kartusche (1) einen abnehmbaren Produktschutz (20) aufweist, wobei der Produktschutz (20) auch als Schutzkappe dient.

## Claims

1. Cartridge droplet dosage device with a cartridge (1) and an actuation element (2), whereby the cartridge (1) comprises a reservoir body (3) with a valve pin (4), a cladding (5) and a plug (6)
**characterized by**
circumferential sealing ribs (12) around a pin diameter and in that
a fluid groove (9) between a wall of the cladding (5) and the valve pin (4) projects from the reservoir body (3) into a inner tube valve area (11), whereby the fluid groove (9) is releasable either by the yielding of the sealing ribs (12) and/or by the yielding of part of the cladding (5).

2. Cartridge droplet dosage device according to claim 1, **characterized in that** the actuation element (2) comprises a piston (8), whereby the piston (8) is actively connectable to the plug (6).

3. Cartridge droplet dosage device according to one of claims 1 to 2, **characterized in that** the circumferential sealing ribs (12) and/or the cladding (5) is flexible under pressure and is resettable.

4. Cartridge droplet dosage device according to one of the previous claims, **characterized in that** the cartridge comprises a bayonet closure (13), whereby the bayonet closure (13) is connectable to a bayonet lock (21) of the actuation element (2).

5. Cartridge droplet dosage device according to one of the previous claims, **characterized in that** the actuation element (2) comprises a perforation area (19), whereby the perforation area (19) is arranged between the piston (8) and a counterpiece of the actuation element (2).

6. Cartridge droplet dosage device according to one of the previous claims, **characterized in that** the piston (8) has a toothing.

7. Cartridge droplet dosage device according to one of the previous claims, **characterized in that** the cartridge (1) has a detachable product protection (20), whereby the product protection (20) also serves as a protective cap.

## Revendications

1. Cartouche de dosage de gouttes avec une cartouche (1) et un élément d'actionnement (2), dans laquelle la cartouche (1) comporte un corps de réservoir (3) avec une goupille de soupape (4), un revêtement (5) et un obturateur (6),
**caractérisée par**
des nervures d'étanchéité circonférentielles (12) autour d'un diamètre d'axe ainsi que par
une rainure à liquide (9) entre une paroi du revêtement (5) et la goupille de soupape (4) qui ressort du corps de réservoir (3) vers une zone de soupape à tuyau flexible (11), où la rainure à liquide (9) peut être libérée soit par la flexion des nervures d'étanchéité (12) et/ou par la flexion d'une partie du revêtement (5).

2. Cartouche de dosage de gouttes selon la revendication 1, **caractérisée par le fait que** l'élément d'actionnement (2) comporte un piston (8), où le piston (8) peut être connecté en fonctionnement à l'obturateur (6).

3. Cartouche de dosage de gouttes selon l'une des revendications 1 à 2, **caractérisée par le fait que** les nervures d'étanchéité circonférentielles (12) et/ou le revêtement (5) sont flexibles à la pression et peuvent être rappelés en position.

4. Cartouche de dosage de gouttes selon l'une des revendications précédentes, **caractérisée par le fait que** la cartouche comporte une fermeture à baïonnette (13), où la fermeture à baïonnette (13) peut être connectée à un verrouillage à baïonnette (21) de l'élément d'actionnement (2).

5. Cartouche de dosage de gouttes selon l'une des revendications précédentes, **caractérisée par le fait que** l'élément d'actionnement (2) comporte une zone de perforation (19), où la zone de perforation (19) est disposée entre le piston (8) et une pièce antagoniste de l'élément d'actionnement (2).

6. Cartouche de dosage de gouttes selon l'une des revendications précédentes, **caractérisée par le fait que** le piston (8) présente une denture.

7. Cartouche de dosage de gouttes selon l'une des revendications précédentes, **caractérisée par le fait que** la cartouche (1) présente une protection de produit amovible (20), où la protection de produit (20) sert également de capuchon de protection.
